# EUROPEAN PATENT SPECIFICATION

(11) **EP 4 221 673 B1**
(45) Date of publication and mention of the grant of the patent: **18.11.2026**
(21) Application number: 21782556.1
(22) Date of filing: 26.09.2021
(51) Int. Cl.: A61K 8/44, A61K 8/49, A61Q 17/00

(54) **A PERSONAL CARE COMPOSITION COMPRISING AMINO ACIDS**
KÖRPERPFLEGEZUSAMMENSETZUNG MIT AMINOSÄUREN
COMPOSITION DE SOINS PERSONNELS COMPRENANT DES ACIDES AMINÉS

(30) Priority: 29.09.2020 EP 20199044
(43) Date of publication of application: 09.08.2023
(73) Proprietor: Unilever IP Holdings B.V., 6708 WH Wageningen (NL); Unilever Global IP Limited, Wirral, Merseyside CH62 4ZD (GB)
(72) Inventor: DASGUPTA, Anindya, Banglaore. Karnataka 560066 (IN); MALLEMALA, Prathyusha, Bangalore, Karnataka 560066 (IN); WASKAR, Morris, Bangalore, Karnataka 560066 (IN)
(74) Representative: Unilever Patent Group
(86) International application number: PCT/EP2021/076426
(87) International publication number: WO 2022/069379

(56) References cited:
- EP-A1- 1 459 736
- WO-A1-2011/109119
- WO-A1-2017/015275
- WO-A1-2020/117755
- CN-B- 107 308 101
- KR-B1- 101 689 878
- US-A1- 2016 089 315
- US-B1- 6 335 004
- DATABASE WPI Week 201801, Derwent World Patents Index; AN 2017-76502P
- UNKNOWN: "Prodew 300-400", 1 January 2013 (2013-01-01), pages 1 - 2, XP055784116, Retrieved from the Internet <URL:https://www.ulprospector.com/documents/1157826.pdf?bs=30&b=213352&st=20&r=na&ind=personalcare> [retrieved on 20210310]

## Description

### Field of the invention

The present invention relates to a personal care composition for protection of skin against undesirable bacteria. The present invention is especially useful in formulating compositions which act as prebiotics for skin commensal bacteria like *S. epidermidis* to produce metabolites which by way of the present invention has been shown to inhibit growth of harmful bacteria like *E. coli, S. aureus* among others. The present invention thus provides for microbiome balancing on the skin.

### Background of the invention

Skin in mammals is considered as the largest organ of the body, and has the largest surface area. Skin forms the first line of defence against microorganisms which may invade the body though the air, water, food or material that come in contact with the body. When the body is infected on the skin or systemically, traditional approach to such hygiene problems has been to treat the skin/ body with antimicrobial actives that reduce or kill the germs. Recent research indicates that a lot of the bacteria that permanently reside on the skin (called skin commensal bacteria) do not normally cause infections in healthy individuals, rather they are beneficial bacteria that protect the skin against disease causing pathogens. Several mechanisms have been proposed to explain the protection and some of the popular ones are: physically occupying space on skin to prevent colonization of pathogens; producing metabolites that ward off harmful, possibly pathogenic organisms; generating metabolites that strengthen the innate defence mechanisms to prevent infection by harmful pathogens; and providing other benefits such as maintaining skin pH, barrier function etc. Thus, of late, there is a trend in moving away from the approach of treating the skin with broad spectrum antimicrobial actives to kill all microoganisms present on skin (or any other part of the body) as a means of treating infections. Rather, the approach is more towards targeted or selective inhibition/ killing of the desired microorganism to the exclusion of the skin commensal organism. This ensures that the skin microbiome is maintained in a healthy balanced state for long term hygiene and health.

The present inventors, in looking to solve the problem of selective kill of non-commensal bacteria on skin like *E.coli* and *S. aureus* directed their research to boosting the number and functionality of skin commensal bacteria through use of prebiotics/Selective Fermentation Inducers (SFI).

The present inventors during the course of their extensive research in the field of prebiotics are aware that many carbon sources e.g carbohydrates and sugars known to be prebiotics for skin, e.g. WO2013/122931 (P&G) discloses topical use of a skin commensal prebiotic to improve the health of the skin microbiome, thereby potentially improving the condition and/or appearance of the skin. In the present invention they explored the use of nitrogen sources (e.g. amino acids) as prebiotics and found to their surprise that only certain select amino acids to the exclusion of others act as prebiotics.

WO 2017/015275 A1 (Quorum Innovations) provides compositions and methods for treating human dermatological conditions by employing a microbiome-centered treatment approach. Preferred embodiments of the invention provide pharmaceutical and cosmetic compositions, and the methods of using the same, comprising a strain of Lactobacillus fermentum bacterium, or bioactive extracts thereof, derived from human microbiota and capable of growing in biofilm phenotype.

It is thus an object of the present invention to provide for a composition that ensures growth of skin commensal bacteria.

It is another object of the present invention to provide for a composition that inhibits growth of harmful microorganisms while ensuring growth of skin commensal bacteria on the skin.

### Summary of the invention

The first aspect of the present invention relates to a personal care composition comprising a combination of amino acids L-Cysteine, L-Serine, L-Alanine, L-Aspartic acid and L- Tyrosine, wherein the composition comprises less than 0.005 wt% total amount of other amino acids.

The second aspect of the present invention relates to a personal care composition comprising a combination of amino acids L-Cysteine, L-Serine and L-Alanine, wherein the composition comprises less than 0.005 wt% total amount of other amino acids.

Another aspect of the present invention relates to a method of nourishing skin commensal bacteria comprising the step of applying a composition of the first aspect on to skin.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The present invention relates to a composition comprising a specific combination of select amino acids for protecting skin against non-commensal bacteria via nourishing skin commensal bacteria. The present inventors have found that if the composition comprises only seven select amino acids, preferably five of them, more preferably only three of them, to the exclusion of other amino acids, the efficacy is better as compared to including one or more or all of the amino acids not in the selected list. Further, it relates to use of this combination in a skin care composition as commensal bacteria nourishing agent. Alternatively, the present invention also relates to use of this combination in a skin care composition as prebiotic for commensal bacterium. The present inventors believe that this is achieved due to only the select amino acids being a prebiotic for a skin commensal bacterium while not being so for the harmful bacterium; and this works only when amino acids not in the select list are absent or at insignificant levels.

Any preference described hereinbelow with regard to one aspect of the invention (e.g. the composition or the use according to the invention) is also preferred for use in any one of the other aspects of the invention.

"Skin" as used herein is meant to include skin on any part of the body (e.g. face, neck, chest, back, arms, underarms, hands, legs, buttocks and scalp). It is also useful for protecting the skin of babies. By babies is meant a child whose age is less than five years preferably less than three years more preferably less than a year. Use of the composition of the invention against pathogens is preferably non-therapeutic, for example, delivered through a cosmetic or personal care composition. Further the composition of the invention and the method of the invention are also preferably for non-therapeutic use. Such a composition could be in the form of a leave-on composition. Alternatively and equally preferably it could be delivered through a wash-off format for delivering selective protective benefit to topical areas e.g. skin and/or hair of mammals, especially humans. Such a composition includes any product applied to a human body for also improving appearance, cleansing, or general aesthetics. The composition of the present invention may be delivered with a topically acceptable carrier which could be an anhydrous base, liquid, lotion, cream, foam, scrub, gel, or emulsion.

The present invention relates to a personal care composition comprising a combination of amino acids L-Cysteine, L-Serine, L-Alanine, L-Aspartic acid and L- Tyrosine, wherein the composition comprises less than 0.005 wt% total amount of other amino acids.

The structure of the amino acids useful as per the present invention and their properties are given in the table - 1 below:

**Table - 1:**

| Amino acid | Three letter code | Structure | Properties |
|---|---|---|---|
| Alanine | Ala | 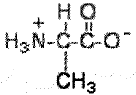 | Neutral Non-polar |
| Aspartic acid | Asp | 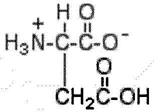 | Acidic Polar |
| Cysteine | Cys | 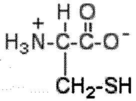 | Neutral Slightly Polar |
| Glycine | Gly | 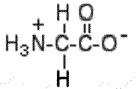 | Neutral Non-polar |
| Serine | Ser | 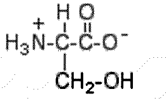 | Neutral Polar |
| Tyrosine | Tyr | 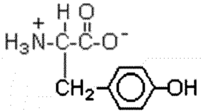 | Neutral Polar |
| Threonine | Thr | 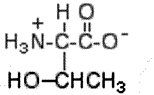 | Neutral Polar |

According to a second aspect of the present invention there is provided a composition comprising a combination of amino acids L-Cysteine, L-Serine and L-Alanine wherein the composition comprises less than 0.005 wt% total amount of other amino acids.

The amino acids selectively included in any aspect of the present invention is preferably present in 0.01 to 1% of each of the amino acids claimed by total weight of the composition. Each of the amino acids in any aspect of the present invention is more preferably included in at least 0.03, even more preferably at least 0.05, further more preferably at least 0.07% and optimally at least 0.1% by weight of the composition. Each of the amino acids in any aspect of the present invention is more preferably included in at most 0.9%, more preferably at most 0.8, further more preferably at most 0.7% and optimally at most 0.5% by weight of the composition. The composition as per the invention includes 0.03 to 7%, preferably 0.05 to 5%, most preferably 0.07 to 3% total amount of amino acids by total weight of the composition.

By "less than 0.05 wt% preferably less than 0.01 wt% other amino acids" is meant that the concentration of each of the amino acids not in the select list as per an aspect of the present invention is less than 0.05% preferably less than 0.01% by weight of the composition. As per an especially preferred aspect of the present invention the concentration of each of the amino acids not in the select list as per an aspect of the present invention is less than 0.005%, most preferred being less than 0.001% by weight of the composition. According to a further preferred aspect the total amount of amino acids not in the select list of an aspect of the present invention is less than 0.05% preferably less than 0.01%, further more preferably less than 0.005% of the total weight of the composition.

According to another preferred aspect of the present invention there is provided a composition comprising less than 1 wt%, preferably less than 0.1 wt% peptides having more than 3 amino acids in its chain. According to yet another preferred aspect of the present invention there is provided a composition comprising less than 1 wt%, preferably less than 0.1 wt% proteins. Thus, the benefit of the present invention is seen when the select amino acids are included in the composition in the absence of peptides or proteins.

The select amino acids present in the composition as per this invention is preferably a prebiotic for a skin commensal bacterium. The skin commensal bacteria is preferably S. *epidermidis.* The skin is protected as per this invention from non-commensal bacteria which may be *E. coli, S. aureus, P aeruginosa,* or mixtures thereof. Consumers often find such non-commensal bacteria to be unwanted or undesirable as they may provide certain negative attributes like producing itchiness, discomfort or malodour. In certain cases such as dry skin, eczema and atopic dermatitis, they may even be harmful or in extreme cases be pathogenic.

The composition of the invention, in one aspect, is preferably a wash-off composition, and this is enabled by including 1 to 80% by weight of a surfactant. In general, the surfactants may be chosen from the surfactants described in well-known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "TensideTaschenbuch", H. Stache, 2nd Edn., Carl Hauser Verlag, 1981. Any type of surfactant, i.e. anionic, cationic, nonionic, zwitterionic or amphoteric can be used but preferred surfactant is of the anionic or non-ionic type.

The pH of a wash off composition as per the present invention is in the range of 4 to 11, preferably in the range of 5.5 to 10.

The surfactant may be a soap. Soap is a suitable surfactant for personal washing applications of composition of the invention. The soap is preferably C8-C24 soap, more preferably C10-C20 soap and most preferably C12-C16 soap. The soap may or may not have one or more carbon-carbon double bond or triple bond. The cation of the soap may be alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils generally used in soap manufacture may be such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc.

A typical fatty acid blend consisted of 5 to 30% coconut fatty acids and 70 to 95% fatty acids ex hardened rice bran oil. Fatty acids derived from other suitable oils/fats such as groundnut, soybean, tallow, palm, palm kernel, etc. may also be used in other desired proportions. The most preferred soap is a laurate soap. The soap, when present in solid forms of the present invention is present in an amount of 30 to 90%, preferably from 50 to 85%, more preferably 55 to 75% by weight of the composition. The soap, when present in liquid forms of the composition is present in 0.5 to 20%, preferably from 1 to 10% by weight of the composition.

Alternatively the surfactants are non-ionic surfactants, such as C8-C22, preferably C8-C16 fatty alcohol ethoxylates, comprising between 1 and 8 ethylene oxide the surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16. Suitable surfactant concentrations in liquid forms of cleaning application are generally more than 0.5% but less than 10%, preferably from 1 to 5 % by weight of the composition. In solid compositions, the surfactant is preferably present in 5 to 40%, preferably from 10 to 30% by weight of the composition.

Water may preferably be present in 10 to 90% by weight of the composition depending on the format of the composition. In solid composition water may be present in 10-30%, while in liquid or semi-solid composition, water may be present in 40 to 90%.

When the composition in accordance with another aspect of the present invention is a leave on composition, it preferably comprises one or more surfactant, emollient, humectant, pigment or preservative.

The pH of a leave on composition as per the present invention is in the range of 5 to 9, preferably in the range of 5.5 to 8.

The carrier acts as diluent or dispersant for the ingredients of the compositions. The carrier may be aqueous-based, anhydrous or an emulsion, whereby a water-in-oil or oil-in-water emulsion is generally preferred. If the use of water is desired, water typically makes up the balance of the composition, which most preferably is from 40 to 80 % by weight of the composition.

In addition to water, organic solvents may optionally be included as carrier to assist any other carrier in the compositions of the present invention. Examples include alkanols like ethyl and isopropyl alcohol.

Other suitable organic solvents include ester oils like isopropyl myristate, cetyl myristate, 2-octyldodecyl myristate, avocado oil, almond oil, olive oil and neopentylglycol dicaprate. Typically, such ester oils assist in emulsifying the compositions, and an effective amount is often used to yield a stable, and most preferably, water-in-oil emulsion.

Emollients may also be used, if desired, as a carrier. Alcohols like 1 -hexadecanol (i.e. cetyl alcohol) are preferred. Other emollients include silicone oils and synthetic esters. Silicone oils suitable for use include cyclic or linear polydimethylsiloxanes containing from 3 to 9, preferably from 4 to 5 silicon atoms. Non-volatile silicone oils useful as emollients include polyalkyl siloxanes, polyalkylaryl siloxanes and polyether siloxane copolymers. The non-volatile polyalkyl siloxanes useful polydimethylsiloxanes. Silicone elastomers may also be used. The ester emollients that may optionally be used are:
(i) alkenyl or alkyl esters of fatty acids having 10 to 20 carbon atoms. Examples thereof include isoarachidyl neopentanoate, isononyl isonanonoate, oleyl myristate, oleyl stearate, and oleyl oleate;
(ii) ether-esters such as fatty acid esters of ethoxylated fatty alcohols;
(iii) polyhydric alcohol esters. Ethylene glycol mono and di-fatty acid esters, diethylene glycol mono- and di-fatty acid esters, polyethylene glycol (200- 6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters;
(iv) wax esters such as beeswax, spermaceti, stearyl stearate and arachidyl behenate; and,
(v) sterols esters, of which cholesterol fatty acid esters are examples.

Emollients, when present, typically make up from 0.1 to 50 % by weight of the composition, including all ranges subsumed therein.

Fatty acids having from 10 to 30 carbon atoms may also be included as carriers. Examples of such fatty acids include pelargonic, lauric, myristic, palmitic, stearic, isostearic, oleic, linoleic, arachidic, behenic or erucic acid and mixtures thereof.

Moisturisation may be improved through use of petrolatum or paraffin. Thickeners may also be utilized as a portion of the carrier in the compositions. Typical thickeners include cross-linked acrylates (e.g. Carbopol^{®} 982), hydrophobically-modified acrylates (e.g. Carbopol^{®}1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.001 to 5, optimally from 0.01 to 0.5 % by weight of the composition.

Surfactants may also be present. When present, the total amount of surfactants is 2 to 40 % by weight, and preferably from 4 to 20 % by weight, optimally from 5 to 12 % by weight of the composition. The surfactant is selected from the group consisting of anionic, nonionic, cationic and amphoteric actives. Particularly preferred nonionic surfactants are those with a C₁₀₋₂₀ fatty alcohol or acid hydrophobe condensed with from 2 to 100 moles of ethylene oxide or propylene oxide per mole of hydrophobe; mono- and di- fatty acid esters of ethylene glycol; fatty acid monoglyceride; sorbitan, mono- and di-C8-C20 fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C_{8 to 20} acyl isethionates, acyl glutamates, C_{8 to 20} alkyl ether phosphates and combinations thereof.

Various other ingredients may also be used in compositions. Actives are defined as skin benefit agents other than emollients and other than ingredients that merely improve the physical characteristics of the composition. Although not limited to this category, general examples include extender pigments such as talcs and silicas, as well as alpha- hydroxy acids, beta-hydroxy acids and zinc salts.

Beta-hydroxy acids include salicylic acid. Zinc oxide and zinc pyrithione are examples of useful zinc salts.

Suitable preservatives include alkyl esters of p-hydroxybenzoic acid, hydantoin derivatives, propionate salts, and a variety of quaternary ammonium compounds. Particularly preferred preservatives are methyl paraben, propyl paraben, phenoxyethanol and benzyl alcohol. Preservatives are from 0.1 to 2 % by weight of the composition.

The packaging could be a patch, bottle, tube, roll-ball applicator, propellant driven aerosol device, squeeze container or lidded jar.

Without wishing to be bound by theory the present inventors believe that many bacteria can synthesize most amino acids using their internal machinery, but may not be able to synthesize all of them. If they don't have the necessary enzymes, they will need them to be supplied from their environment. Also, they always prefer using amino acids already available in their environment rather than synthesise them as it requires energy to make them. In the current scenario, we identified a critical mixture of amino acids needed to support good growth of commensal bacterium, but fortuitously they also did not provide any benefit to the pathogenic bacterium..

The present invention is therefore intended to increase, promote, improve, maintain or sustain skin health and skin resiliency. This is attained through ensuring a healthy skin ecosystem through balanced microbiome health. By balanced microbiome health is meant that the ratio of commensal bacteria to non-commensal bacteria is maintained in a desired range for the skin health to be maintained.

This invention is especially useful for use on skin of babies where the use of broad spectrum anti-bacterial agents are considered too harsh as it tends to interfere with the growth and maintenance of a healthy skin microbiome for long term health of the babies as they grow into adults. Thus, whether used on skin of babies or others, the composition for use in the present invention is substantially free of a conventional antimicrobial compound. By substantially free is meant that conventional antimicrobial actives are present in less than 0.1wt%, more preferably less than 0.05 wt%, further more preferably less than 0.01wt%, even more further preferably less than 0.001% by weight of the composition. By conventional antimicrobial actives is meant an antimicrobial active which kills or inhibits bacteria which attack skin like *E. coli, S. aureus, P aeruginosa,* among others. Preservatives which are included in compositions for their microbial stability are excluded from the definitions of antimicrobial actives mentioned above. Preservatives are included to ensure that the compositions are stable with respect to microorganisms which may grow and degrade the compositions. On the other hand, antibacterial compounds are included in compositions to hinder the growth of microorganisms which are present on the substrate (e.g skin) on which the compositions are applied.

Conventional antimicrobial compounds are generally those from the class of biguanides, bisphenols, halophenols, oligodynamic metal compounds like those of silver or zinc, cationic antimicrobial compounds or essential oil actives. Biguanide has a general base structure which may be further derivatised e.g. chlorhexidine or polyhexamethylene biguanide (PHMB). Bisphenols include triclosan or hexachlorophene. Halophenol include chloroxylenol (PCMX). Cationic compounds are another class of antimicrobial actives e.g. benzalkonium chloride, cetyl pyridinium chloride or cetyl trimethyl ammonium bromide.

The class of low boiling alcohols (which are fast acting antimicrobials) are especially not included in any substantial amount in the composition of the invention. Ideally, they are absent from such compositions. By low boiling alcohols are meant monohydric alcohols with 2 to 5 carbon atoms.

When the composition of the invention is formulated for use on skin of babies, it is preferred that it is substantially free of a fragrance component. By a fragrance component is meant a molecule or a group of molecules that are compounded to form a perfume. The characteristic of such compounds is that they are volatile (to varying degrees) and offer a pleasant odour. In the context of perfumes or fragrance component, "by substantially free" is meant that that they are present in less than 0.05wt%, more preferably less than 0.01 wt%, further more preferably less than 0.005wt%, even more further preferably less than 0.001% by weight of the composition.

In another aspect the present invention relates to use of a composition comprising a combination of amino acids L-Cysteine, L-Serine, L-Alanine, L-Aspartic acid and L-Tyrosine wherein the composition comprises less than 0.005 wt% total amount of other amino acids. The use according to the invention is of a non-therapeutic nature.

Yet another aspect of the present invention relates to a method of nourishing skin commensal bacteria while inhibiting the growth of harmful bacteria comprising the step of applying a composition of the present invention, on to skin. They have observed this with experimental data on skin commensal bacteria S. *epidermidis* but it is likely possible with many other such beneficial bacteria known to colonise healthy skin. The method of the invention is effective in inhibiting harmful bacteria which comprises one or more selected from *E. coli, S. aureus, P. aeruginosa* or mixtures thereof.

The invention will now be demonstrated with the help of the following non-limiting examples.

### Examples

### Example -1: Growth of S. epidermidis in the presence of a single amino acid as supplement:

The growth of the above commensal bacteria due to the presence of each of the twenty different amino acids separately was studied. All 20 L- amino acids were procured from Hi media. The stocks were prepared in distilled water and filter sterilized by passing it through 0.22 µm filter. A 100 mM sodium phosphate buffer pH 7.4 was prepared and filter sterilized. Reaction was set up in a 96 well flat bottom transparent plate. 100 µl of 20% TSB (prepared by diluting 100% TSB in 100 mM sodium phosphate buffer) was added to respective wells followed by addition of 20 µl of 10% glucose (prepared in water and filter sterilized), 20 µl of 1% individual amino acid stock (in control wells instead of amino acid stock same volume of buffer was added), 20 µl of 0.2 OD₆₀₀ₙₘ *S .epidermidis* culture (22-24 hrs plate culture) corresponding to 10⁸ CFU/ml prepared in 100 mM sodium phosphate buffer and the final volume was made up to 200 µl with buffer. The final concentration of individual amino acid was 0.1%. Each reaction was set up in triplicates. The plate was incubated at 37°C under stationary conditions for 24 hrs. After 24 hrs, the absorbance of the plate was measured at 600 nm using TECAN plate reader. The absorbance values were then normalized to control *(S. epidermidis* grown in 10% TSB+1% glucose). The data on the % growth of each of the samples with respect to the control is summarized in Table - 1 below:

**Table - 1**

| Example | Amino acid | Three letter code | % growth |
|---|---|---|---|
| 1A | Alanine | Ala | 139 |
| 1B | Arginine | Arg | 95 |
| 1C | Asparagine | Asn | 113 |
| 1D | Aspartic acid | Asp | 121 |
| 1E | Cysteine | Cys | 121 |
| 1F | Glycine | Gly | 135 |
| 1G | Glutamic acid | Glu | 110 |
| 1H | Glutamine | Gln | 109 |
| 1I | Histidine | His | 96 |
| 1J | Isoleucine | Ile | 99 |
| 1K | Leucine | Leu | 96 |
| 1L | Lysine | Lys | 85 |
| 1M | Methionine | Met | 87 |
| 1N | Phenyl alanine | Phe | 100 |
| 1O | Proline | Pro | 93 |
| 1P | Serine | Ser | 134 |
| 1Q | Tyrosine | Tyr | 120 |
| 1R | Threonine | Thr | 126 |
| 1S | Tryptophan | Trp | 99 |
| 1T | Valine | Val | 96 |

The sample in the above table having a higher absolute value is to be interpreted as an amino acid which ensures higher growth of skin commensal bacteria S. *epidermidis.*

### Example - 2: Growth of S. epidermidis is in the presence of 0.2% amino acid mixture (of all twenty amino acids with a single amino acid depletion)

Twenty different amino acids mixes were prepared by mixing equal proportions of all individual amino acid stocks excluding one amino acid in each mix (equal volume of water was added instead of excluded amino acid). Control amino acid mix was prepared by mixing equal proportions of all 20 individual amino acid stocks.

Reaction was set up in a 96 well flat bottom transparent plate. 100 µl of 20% TSB (prepared by diluting 100% TSB in 100 mM sodium phosphate buffer) was added to wells followed by addition of 20 µl of 10% glucose (prepared in water and filter sterilized), 20 µl of 2% amino acid mix, 20 µl of 0.2 OD₆₀₀ₙₘ S. *epidermidis* culture (22-24 hrs plate culture) corresponding to 10⁸ CFU/ml prepared in 100 mM sodium phosphate buffer pH 7.4 and the final volume was made up to 200 µl with buffer. Each reaction was set up in triplicates. Final concentration of individual amino acid in the mix is 0.01%.

The plate was incubated at 37°C under stationary conditions for 24 hrs. After 24 hrs, the absorbance of the plate was measured at 600 nm using TECAN plate reader. The absorbance values were then normalized to control *(S. epidermidis* grown in 10% TSB+1% glucose+0.2% amino acid mix containing all 20 amino acids).

The data on the % growth of each of the samples with respect to the control is summarized in Table - 2 below. The amino acid indicated in each of the rows below indicates the amino acid which was not included in that particular sample:

**Table - 2**

| Example | Amino acid | Three letter code | % growth |
|---|---|---|---|
| 2A | Alanine | Ala | 94 |
| 2B | Arginine | Arg | 90 |
| 2C | Asparagine | Asn | 92 |
| 2D | Aspartic acid | Asp | 88 |
| 2E | Cysteine | Cys | 77 |
| 2F | Glycine | Gly | 101 |
| 2G | Glutamic acid | Glu | 102 |
| 2H | Glutamine | Gln | 92 |
| 2I | Histidine | His | 89 |
| 2J | Isoleucine | Ile | 99 |
| 2K | Leucine | Leu | 92 |
| 2L | Lysine | Lys | 94 |
| 2M | Methionine | Met | 100 |
| 2N | Phenyl alanine | Phe | 101 |
| 2O | Proline | Pro | 99 |
| 2P | Serine | Ser | 80 |
| 2Q | Tyrosine | Tyr | 94 |
| 2R | Threonine | Thr | 97 |
| 2S | Tryptophan | Trp | 104 |
| 2T | Valine | Val | 105 |

The sample in the above table having a lower absolute value is to be interpreted as an amino acid which ensures higher growth of skin commensal bacteria S. *epidermidis.* Thus the lower value the better.

Based on the (difference between) the data in Table - 1 and Table - 2 above, the present inventors shortlisted the amino acids most expected to ensure growth of S. *epidermidis* to be seven of the amino acids namely: L-Cysteine, L-Serine, L-Alanine, L-Aspartic acid, L- Tyrosine, L- Glycine, and L-Threonine.

### Example - 3: Growth pattern of S. epidermidis/S. aureus in the presence of a mixture of the above seven amino acids.

Growth kinetics experiments were carried out for both *S. epidermidis* (commensal) and *S. aureus* (pathogen) in the presence of amino acid mix (Mix 7) which was prepared using a set of seven amino acids (Alanine, Aspartic acid, Cysteine, Glycine, Serine, Tyrosine and Threonine) which were shown to boost the growth of *S.epidermidis* in supplementation experiments. The growth kinetics of the above two micro-organisms measured as absorbance at 600 nm is shown in table 3 below.

**Table - 3: Growth kinetics of S. epidermidis and S. aureus: 10% TSB + 1% glucose + 0.1% amino acid mix (Mix 7)**

| Time (hrs) | *S. Epidermidis* Absorbance at 600 nm | *S. aureus* Absorbance at 600 nm |
|---|---|---|
| 6 | 0.264 | 0.324 |
| 10 | 0.494 | 0.694 |
| 14 | 0.706 | 0.920 |
| 18 | 0.890 | 0.890 |
| 20 | 1.012 | 0.870 |

The data in the above table indicates that the mix of the seven selected amino acids ensures continuous growth of S. *epidermidis* even after 18 hours while the growth of S. *aureus* starts to reduce after 18 hours.

### Example - 4: Identification of the five most effective amino acids that stunts the growth of S. aureus from the selected seven

In order to identify a set of amino acids which are major contributors to the higher growth rate of S. *aureus* in Mix 7, growth kinetics experiments were carried out with all seven individual amino acids. The kinetic data showed monotonic increase with plateauing of growth for all the seven amino acids. The experiment was carried out for growth till 20 hours. They were carried out at 0.1wt% concentration of the individual amino acid. The data in terms of absorbance at 600nm is summarized in Table - 4 below:

**Table - 4:**

| Example | Amino Acid | Absorbance at 600 nm |
|---|---|---|
| 4A | Alanine | 0.447 |
| 4B | Cysteine | 0.476 |
| 4C | Tyrosine | 0.478 |
| 4D | Serine | 0.504 |
| 4E | Aspartic Acid | 0.540 |
| 4F | Threonine | 0.697 |
| 4G | Glycine | 0.863 |

The data in the table -4 above indicates that the first five amino acids viz. Cysteine, Serine, Alanine, Aspartic acid and Tyrosine ensure maximum inhibition of *S.aureus* which is what is desired.

### Example - 5: Growth pattern of S. epidermidis/S. aureus in the presence of a mixture of the select five amino acids.

Growth kinetics experiments were carried out for both *S. epidermidis* and *S. aureus* in the presence of amino acid mix (Mix 5) which was prepared using a set of five selected amino acids (Alanine, Aspartic acid, Cysteine, Serine, and Tyrosine) which were identified in the experiment above. The growth kinetics of the above two micro-organisms measured as absorbance at 600 nm are shown in table 5 below.

**Table - 5: Growth kinetics of S. epidermidis and S. aureus. in the presence of 10% TSB, 1% glucose and 0.1% amino acid mix (with Mix 5)**

| Time (hrs) | *S. Epidermidis* Absorbance at 600 nm | *S. aureus* Absorbance at 600 nm |
|---|---|---|
| 6 | 0.228 | 0.283 |
| 10 | 0.502 | 0.401 |
| 14 | 0.571 | 0.456 |
| 18 | 0.712 | 0.486 |
| 20 | 0.750 | 0.499 |

The data in the above table indicates that the mix of the five selected amino acids ensures continuous growth of S. *epidermidis* while the growth of S. *aureus* is much lower. Thus, the desired behaviour of microbiome balance is significantly observed with the mix of these five amino acids.

### Example - 6: Growth pattern of S. epidermidis/S. aureus in the presence of smaller subsets of the Mix 5

To further optimize and minimize the amino acid mix from the selected mixture of five amino acids which can preferentially support the growth of S. *epidermidis* over S. *aureus,* growth kinetics experiments were carried for both *S. epidermidis* and S. *aureus* in the presence of different amino acid combinations containing two, three or four amino acids keeping the two essential amino acids *i.e.,* Cysteine and Serine as the common ones) in all the combinations tested. These two were selected as their individual absence led to the lowest growth from the various combinations of amino acids as seen from Table - 2. The data is summarized in table - 6 below:

**Table 6: Growth of S. epidermidis and S. aureus in the presence of amino acid mix (10% TSB+1% glucose+0.1% amino acid mix) in comparison to its control (10% TSB+1% glucose)**

| Amino acid mix tested | Composition | *S. epidermidis* growth in comparison to its control | *S. aureus* growth in comparison to its control |
|---|---|---|---|
| Mix 2 | Cys, Ser | - | - |
| Mix 3a | Cys, Ser, Ala | + | - |
| Mix 3b | Cys, Ser, Asp | - | - |
| Mix 3c | Cys, Ser, Tyr | - | + |
| Mix 4a | Cys, Ser, Ala, Asp | + | - |
| Mix 4b | Cys, Ser, Ala, Tyr | + | - |
| Mix 4c | Cys, Ser, Asp, Tyr | - | - |

| | | | |
|---|---|---|---|
| Note: "-"- indicates growth similar to its control; "+" - indicates higher growth compared to control | | | |

The data in the table - 6 above indicates that the smallest subset which gives the desired result i.e. higher growth of S. *epidermidis* and lower growth of S. *aureus* is Mix 3a which is combination of Cysteine, Serine and Alanine.

### Example - 7: Growth pattern of S. epidermidis/S. aureus in the presence of a mixture of the select three amino acids.

Growth kinetics experiments were carried out for both *S. epidermidis* and *S. aureus* in the presence of amino acid mix (Mix 3a) which was prepared using a set of three selected amino acids (Alanine, Cysteine, and Serine,) which were identified in the experiment above. The growth kinetics of the above two micro-organisms measured as absorbance at 600 nm are shown in table 7 below.

**Table - 7: Growth kinetics of S. epidermidis and S. aureus in the presence of 10% TSB+1% glucose+0.1% amino acid mix (Mix 3a)**

| Time (hrs) | *S. Epidermidis* Absorbance at 600 nm | S. *aureus* Absorbance at 600 nm |
|---|---|---|
| 12 | 0.477 | 0.424 |
| 18 | 0.670 | 0.481 |
| 24 | 0.816 | 0.512 |

The data in the above table indicates that the mix of the three selected amino acids ensures continuous growth of S. *epidermidis* while the growth of S. *aureus* is much lower. Thus, significant microbiome balance is observed with the optimized mix of these three amino acids.

## Claims

1. A personal care composition comprising a combination of amino acids L-Cysteine, L-Serine, L-Alanine, L-Aspartic acid and L- Tyrosine, wherein the composition comprises less than 0.005 wt% total amount of other amino acids.

2. A personal care composition comprising a combination of amino acids L-Cysteine, L-Serine and L-Alanine, wherein the composition comprises less than 0.005 wt% total amount of other amino acids.

3. A composition as claimed in claims 1 or 2 comprising 0.01 to 1 wt% of each of the amino acids claimed.

4. A composition as claimed in any one of the preceding claims comprising 0.07 to 7 wt% total amount of amino acids.

5. A composition as claimed in any one of the preceding claims comprising less than 1 wt%, preferably less than 0.1 wt% peptides having more than 3 amino acids in its chain.

6. A composition as claimed in any one of the preceding claims comprising less than 1 wt% preferably less than 0.1 wt% proteins.

7. A method of nourishing skin commensal bacteria comprising the step of applying a composition as claimed in any one of preceding claims on to skin,
wherein the method is non-therapeutic.

8. A method as claimed in claim 7 wherein the skin commensal bacteria is S. *epidermidis,*
wherein the method is non-therapeutic.

9. A method of nourishing skin commensal bacteria while inhibiting the growth of harmful bacteria comprising the step of applying a composition as claimed in any one of the preceding claims 1 to 6, on to skin,
wherein the method is non-therapeutic.

10. A method as claimed in claim 9 wherein the harmful bacteria comprise *E. coli, S. aureus, P. aeruginosa,* or mixtures thereof,
wherein the method is non-therapeutic.

11. Use of a composition comprising a combination of amino acids L-Cysteine, L-Serine, L-Alanine, L-Aspartic acid and L- Tyrosine, wherein the composition comprises less than 0.005 wt% total amount of other amino acids for providing microbiome benefit to skin,
wherein the use is non-therapeutic.

## Patentansprüche

1. Körperpflegezusammensetzung, die eine Kombination der Aminosäuren L-Cystein, L-Serin, L-Alanin, L-Asparaginsäure und L-Tyrosin umfasst, wobei die Zusammensetzung eine Gesamtmenge anderer Aminosäuren von weniger als 0,005 Gew.-% umfasst.

2. Körperpflegezusammensetzung, die eine Kombination der Aminosäuren L-Cystein, L-Serin und L-Alanin umfasst, wobei die Zusammensetzung eine Gesamtmenge anderer Aminosäuren von weniger als 0,005 Gew.-% umfasst.

3. Zusammensetzung nach Anspruch 1 oder 2, die 0,01 bis 1 Gew.-% von jeder der beanspruchten Aminosäuren umfasst.

4. Zusammensetzung nach irgendeinem der vorhergehenden Ansprüche, die eine Gesamtmenge an Aminosäuren von 0,07 bis 7 Gew.-% umfasst.

5. Zusammensetzung nach irgendeinem der vorhergehenden Ansprüche, die weniger als 1 Gew.-%, vorzugsweise weniger als 0,1 Gew.-% Peptide mit mehr als 3 Aminosäuren in ihrer Kette umfasst.

6. Zusammensetzung nach irgendeinem der vorhergehenden Ansprüche, die weniger als 1 Gew.-%, vorzugsweise weniger als 0,1 Gew.-% Proteine umfasst.

7. Verfahren zur Ernährung kommensaler Bakterien der Haut, umfassend den Schritt des Aufbringens einer Zusammensetzung nach irgendeinem der vorhergehenden Ansprüche auf die Haut, wobei das Verfahren nicht-therapeutisch ist.

8. Verfahren nach Anspruch 7, wobei es sich bei den kommensalen Bakterien der Haut um S. epidermidis handelt, wobei das Verfahren nicht-therapeutisch ist.

9. Verfahren zur Ernährung kommensaler Bakterien der Haut bei gleichzeitiger Hemmung des Wachstums schädlicher Bakterien, umfassend den Schritt des Aufbringens einer Zusammensetzung nach irgendeinem der vorhergehenden Ansprüche 1 bis 6 auf die Haut, wobei das Verfahren nicht-therapeutisch ist.

10. Verfahren nach Anspruch 9, wobei die schädlichen Bakterien E. coli, S. aureus, P. aeruginosa oder Mischungen davon umfassen, wobei das Verfahren nicht-therapeutisch ist.

11. Verwendung einer Zusammensetzung, die eine Kombination der Aminosäuren L-Cystein, L-Serin, L-Alanin, L-Asparaginsäure und L-Tyrosin umfasst, wobei die Zusammensetzung eine Gesamtmenge anderer Aminosäuren von weniger als 0,005 Gew.-% umfasst, um einen Nutzen für das Hautmikrobiom bereitzustellen, wobei die Verwendung nicht-therapeutisch ist.

## Revendications

1. Composition de soins personnels comprenant une combinaison d'acides aminés L-cystéine, L-sérine, L-alanine, acide L-aspartique et L-tyrosine, ladite composition comprenant moins de 0,005 % en poids de la quantité totale d'autres acides aminés.

2. Composition de soins personnels comprenant une combinaison d'acides aminés L-cystéine, L-sérine et L-alanine, ladite composition comprenant moins de 0,005 % en poids de la quantité totale d'autres acides aminés.

3. Composition selon la revendication 1 ou la revendication 2, comprenant 0,01 à 1 % en poids de chacun des acides aminés revendiqués.

4. Composition selon l'une quelconque des revendications précédentes, comprenant 0,07 à 7 % en poids de la quantité totale d'acides aminés.

5. Composition selon l'une quelconque des revendications précédentes, comprenant moins de 1 % en poids, de préférence moins de 0,1 % en poids de peptides ayant plus de 3 acides aminés dans leur chaîne.

6. Composition selon l'une quelconque des revendications précédentes, comprenant moins de 1 % en poids, de préférence moins de 0,1 % en poids de protéines.

7. Procédé de nutrition des bactéries commensales de la peau, comprenant l'étape consistant à appliquer une composition selon l'une quelconque des revendications précédentes sur la peau, ledit procédé étant non thérapeutique.

8. Procédé selon la revendication 7, dans lequel la bactérie commensale de la peau est S. *epidermidis*, ledit procédé étant non thérapeutique.

9. Procédé de nutrition des bactéries commensales de la peau tout en inhibant la croissance de bactéries nocives, comprenant l'étape consistant à appliquer une composition selon l'une quelconque des revendications 1 à 6 sur la peau, ledit procédé étant non thérapeutique.

10. Procédé selon la revendication 9, dans lequel les bactéries nocives comprennent *E*. *coli, S. aureus, P. aeruginosa* ou des mélanges de celles-ci, ledit procédé étant non thérapeutique.

11. Utilisation d'une composition comprenant une combinaison d'acides aminés L-cystéine, L-sérine, L-alanine, acide L-aspartique et L-tyrosine, ladite composition comprenant moins de 0,005 % en poids de la quantité totale d'autres acides aminés, pour apporter un bénéfice microbiomique à la peau, ladite utilisation étant non thérapeutique.
